# EUROPEAN PATENT APPLICATION

(11) **EP 3 854 457 A1**
(43) Date of publication of application: **28.07.2021**
(21) Application number: 20152943.5
(22) Date of filing: 21.01.2020
(51) Int. Cl.: A61Q 11/00, A61K 8/19, A61K 8/22

(54) **TOOTH WHITENING COMPOSITION AND DEVICE FOR DELIVERING THE SAME**

(71) Applicant: Université de Genève, 1211 Genève (CH)
(72) Inventor: KREJCI, Ivo, 1291 Commugny (CH); ARDU, Stefano, 1206 Genève (CH)
(74) Representative: KATZAROV S.A.

(57) **Abstract**

The present invention relates to a tooth whitening composition delivery system comprising a plurality of compartments adapted to house at least one tooth whitening agent and at least one additional substance chosen in the group comprising a self-warming composition and a doping substance, in different compartments, said compartments being separated by a separation module, wherein the system is adapted to suppress said separation provided by the separation module upon activation, thereby permitting mixing of the tooth whitening agent and the at least one additional substance.

## Description

### Technical Field

The present invention relates to tooth whitening compositions and more particularly to tooth whitening compositions with enhanced whitening activity and to a device specifically adapted to deliver tooth whitening compositions with enhanced whitening activity to the teeth of a user.

### Background of the art

Nowadays, tooth whitening has become one of the most frequently requested dental procedures by the public, and hence represents an important market worldwide. However, the public already has access to many different treatments including home-based products such as toothpastes, gels, and films, as well as in-office based systems where products containing highly concentrated whitening agents are applied under professional supervision. It is therefore reasonable to allege that tooth whitening has become a very popular esthetic procedure worldwide.

In order to carry out such a whitening procedure, the standard method is to apply materials comprising whitening compositions directly to the teeth. The standard materials used for this procedure are gels containing H2O2. Alternatively, gels containing carbamide peroxide which release peroxide can be found on the market.

For professional use, high concentrations of carbamide peroxide of around 15 to 20% as well as of 6 to 40% H2O2 are offered by the manufacturers. These highly concentrated gels allow for a good efficiency. However, in 2011, the European legislation limited the concentration of the whitening gels to 6% H2O2 for professional use, which corresponds to 18% carbamide peroxide.

For over-the-counter products, i.e. products which can freely be purchased by anybody, the concentration of H2O2 in the whitening compositions was even more limited to max. 0.1%.

In parallel, due to the increasing criticism of H2O2-containing whitening products, H2O2-free whitening agents have also been introduced on the market. However, low concentration H2O2 and carbamide peroxide and H2O2 free whitening agents such as PAP etc. seriously suffer of low whitening activity.

This is why it is of importance to find ways to enhance the whitening activity of these substances while limiting the H2O2 and carbamide peroxide concentration below the EU legislation requirements.

The current whitening compositions use conventional tooth bleaching techniques which are based on the release of reactive forms of oxygen (such as hydroxyl radical) that reduce complex carbonic chains of pigments into non-colored smaller molecules, hence lightering teeth, and which are well-known techniques and are commonly used for in-office and take-home dental treatments.

These oxidizing techniques often involve Fenton reactions, where H2O2 is delivered as hydrogen peroxide or carbamide peroxide.

Fe 2+ + H2O2 → Fe 3+ + OH - + OH

Fe 3+ + H2O2 → Fe 2+ + OOH + H+

It is well known that these reactions can be activated and boosted with different types of light thereby provoking a photo-catalysis. Furthermore, it is well known that heat increases the activity of tooth whitening gels thereby provoking a thermo-catalysis. In a more general view, the speed at which reactants are converted into products (i.e., reaction rate) depends on the temperature and can be described using the Arrhenius equation (the higher the temperature, the higher the reaction rate).

However, it is very complicated to provide light and/or heat in the buccal cavity once the mouth is closed with the gel inside. Therefore, there exists a need for whitening agents suitable for home and/or professional in-office use whose activity is enhanced without the supply of energy from the outside of the mouth.

Delivery devices integrating LED lights and/or an electric activation system are known and/or already commercialized, however they are expensive and do not provide real enhancement of the whitening activity.

In this regard, a primary object of the invention is to solve the above-mentioned problems and more particularly to provide a way to enhance the activity of whitening agents with low efficiency such as low concentration H2O2 whitening agents, i.e. below or equal to 6% H2O2 for professional use and below or equal to 0.1% H2O2 for OCT, as well as H2O2-free whitening agents such as PAP-containing whitening gels.

### Summary of the invention

The above problems are solved by the present invention which is a way to enhance the activity of whitening agents with low efficiency such as low concentration H2O2 whitening agents, i.e. below or equal to 6% H2O2 for professional use and below or equal to 0.1% H2O2 for OCT, as well as H2O2-free whitening agents such as PAP-containing whitening gels.

Chemical reactions such as tooth whitening procedures follow the temperature rule of doubling the reaction efficiency by increasing the temperature by 10°C, the basic principle of the invention is to introduce self-warming options which allow to increase the temperature of the whitening agents from roughly 30°C present on the surface of teeth to around 40°C, without the need of an input of energy by external energy sources such as light, electricity or mechanical energy.

According to the present invention, the temperature of self-warming whitening agents is controlled by the addition of regulating substances such as sodium acetate or a mixture of vinegar and sodium bicarbonate or the same provided that they produce heat by exothermic reactions and occurs directly inside of the whitening agent or within the delivery device which accepts the whitening agent.

The present invention therefore relates to a tooth whitening composition comprising tooth whitening agents selected in the group comprising H2O2, carbamide peroxide, PAP or the same, and chemical compounds which enhance the whitening activity of low concentration H2O2 by addition of doping substances such as iron particles, or even chelated iron, and/or rejuvenating agents and comprising chemical compounds such as sodium acetate or a mixture of vinegar and sodium bicarbonate or the same provided that they produce heat by exothermic reactions, to allow for a temporary self-warming of the whitening composition or others in the mouth of the patient without the supply of energy from the outside of the mouth.

As mentioned above, although it is not limited to it, the present invention is specially designed for (1) gels that contain low concentrations of H2O2 or carbamide peroxide or (2) gels that are free of H2O2 and contain PAP.

Preferably, the present invention consists in the addition of a self-warming substance to the whitening composition that can undergo exothermic reaction so as to increase the temperature of the whitening gels by approximately 10°C, for example from about 30°C to 40°C.

The self-warming substance can be incorporated directly into the gels but according to a preferred embodiment of the invention is placed in a whitening composition delivery system, preferably a dental tray, which comprises a plurality of compartments arranged so as to house the whitening agent and the self-warming substance in different compartments.

Thanks to this arrangement, the self-warming substance and the whitening composition can be mixed together at a chosen time, such as when the user inserts the dental tray in the mouth and bites (or applies any type of pressure to) the tray so as to break the separation of the compartments and thereby permits the mixing.

In this preferred embodiment, the arrangement of the compartment may be chosen so as to maximize the mixing, for example by stacking the two compartments on each other in the vertical direction, where vertical direction means the direction between the upper and lower jaws once the dental tray is in the biting position.

Advantageously, the compartments can be provided along the whole dental tray or along a specific portion of it, if only specific teeth require whitening procedure.

Alternatively, the delivery system can be a dedicated syringe, also comprising several compartments separating the whitening agent from the self-warming substance and allowing mixing upon delivery either directly to the teeth surface or optionally in a mixing chamber disposed between the outlet of the syringe and the different compartments.

According to a preferred embodiment, the number of compartments in the delivery system is not limited since even the reagent of the self-warming substance can be placed in different compartments or even the reagents of the whitening agents can be housed in different compartments, and/or the doping substances can be housed in different compartments. This permits to use reagents having a very high reaction kinetic. Also, the self-warming substances contained in the delivery device can be activated on purpose by biting or any other kind of pressure, with or without a nucleation appliance.

Advantageously, the compartments can be arranged so as to provide a predetermined sequence of mixing step. This can for example consist in providing separation membranes between the compartments within a dental tray which have different resistances so as to permit breaking specific ones before other ones etc.

In addition to the self-warming substances, the present invention also comprises the possibility to add doping substances to the gels, such as iron particles possibly coated with chelating agents for Fenton reaction. Such doping substances can also be provided in a separated compartment within the delivery system.

Preferably, the delivery device can also include LED lights and/or ultrasonic generators to boost the reactions.

According to the present invention, the whitening composition may further comprise hyaluronic acid, preferably hydrolyzed hyaluronic acid, and/or hypochlorite acid to improve the penetration into the biofilm, reduce the recolonization of bacteria to reform the biofilm and to have a regenerating effect on gingival tissues. This hyaluronic acid and or hypochlorite acid can be housed in a separated compartment or not.

Advantageously, it further comprises remineralizing and/or desensibilizing agents such as CPP/ACP, amorphous calcium phosphate, potassium nitrate or fluoride.

According to the present invention, the delivery system is made for one jaw (upper or lower) or for both jaws (upper and lower) simultaneously.

Preferably, the delivery device is manufactured as customized such as a prefabricated tray or based on a classic or optical impression and produced manually, for example dental-lab produced individual bleaching trays, or by mass customization technology (e.g. 3D printing).

In addition, one can boost the whitening activity by ultrasonic warming between 25 and 50°C, more particularly between 30° and 40°C, applied by appropriate piezo elements which are contained in the delivery system. Preferably the piezo elements can be controlled through detection of the biting.

A preferred embodiment comprises a delivery device of the present invention with a gel or a liquid containing a combination of PAP or PAP based mixtures in concentrations between 10 and 20%, preferably 14 and 17%, even more particularly 15%.

According to a preferred embodiment the delivery system of the present invention is adapted to contain at least one of the substances comprised in the group of sodium hyaluronate at concentrations of 5% or lower, Aloe Vera at concentrations of 5% or lower, sugar alcohols such as Xylitol, Sorbitol, Erythritol to the gel/liquid, cranberry seed oil at concentrations of 5% or lower, Chamomile at concentrations of 5% or lower, baking soda to the gel/liquid at concentrations of 5% or lower, and stable ozonized oils made out of unsaturated plant acids at concentrations of 5% or lower.

According to a preferred embodiment, the self-warming composition is the product of an exothermic reaction. Preferably, the reagents of said exothermic reaction are contained in at least two separated compartments of said tooth whitening composition delivery system such that the reaction is carried out upon activation at the same time when the whitening agent is applied to the tooth/teeth.

The separation module is not particularly limited to any form provided that it hermetically separates the compartments and is adapted to suppress this separation upon activation. For example, the separation module separating at least two compartments can be at least one of a separation membrane adapted to be torn upon biting and of a mobile languet connected to a stirrer wherein the stirrer is adapted to stir the reagents contained of said at least two compartments upon displacement of said languet.

Here as the preferred embodiment, the stirrer is at least one of a helix, spiral and a worm screw made of a proper material.

### Brief description of the drawings

Further particular advantages and features of the invention will become more apparent from the following non-limitative description of at least one embodiment of the invention which will refer to the accompanying drawings, wherein
- Figure 1 schematically represents a tooth whitening composition delivery system according to a first embodiment of the invention;
- Figure 2 schematically represents a tooth whitening composition delivery system according to a second embodiment of the invention;
- Figure 3 schematically represent a cross section of a tooth whitening composition delivery system according to a third embodiment of the invention;
- Figure 4 schematically represent a tooth whitening composition delivery system according to a fourth embodiment of the invention;

### Detailed description of the invention

The present detailed description is intended to illustrate the invention in a non-limitative manner since any feature of an embodiment may be combined with any other feature of a different embodiment in an advantageous manner.

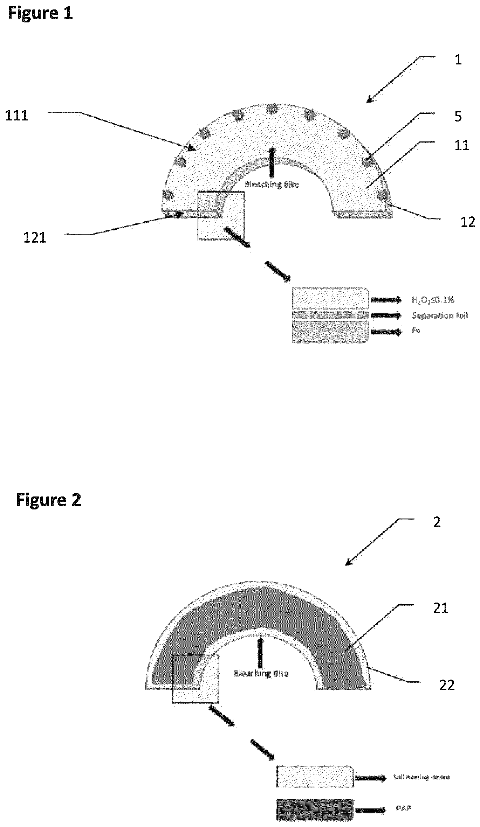

Figure 1 schematically represents a tooth whitening composition delivery system 1 according to a first embodiment of the invention where the delivery system is a dental tray and comprises a plurality of compartments 11, 12 adapted to house at least one tooth whitening agent 111, here H2O2 preferably below 0.1%, and at least one additional substance 121. As per the possible additional substance, it can be at least one or more self-warming composition and one or more doping substance such as iron like in figure 1, chelated iron, and rejuvenating agents.

Furthermore, the additional substance can be at least one of sodium hyaluronate at concentrations of 5% or lower, Aloe Vera at concentrations of 5% or lower, sugar alcohols such as Xylitol, Sorbitol, Erythritol to the gel/liquid, cranberry seed oil at concentrations of 5% or lower, Chamomile at concentrations of 5% or lower, baking soda to the gel/liquid at concentrations of 5% or lower, and stable ozonized oils made out of unsaturated plant acids at concentrations of 5% or lower.

Also, the system can further house at least one of hyaluronic acid, remineralizing and/or desensibilizing agents such as CPP/ACP, amorphous calcium phosphate, potassium nitrate or fluoride.

In the figure 1, which is very schematic and does represent the function and the basic features of the device more than its real structure, two compartments comprising a specific substance, one tooth whitening agent 111 and at least one additional substance 121, are shown, which are stacked on each other on the vertical direction. As shown by the arrow, the vertical direction here means the direction between the upper and lower jaws once the dental tray is in the biting position.

The two compartments are separated by a separation module shown in the magnification portion of figure 1. In the first embodiment, the separation module separates two compartments but the invention is in no manner limited to separating only two compartments and could separate more than two. On the other hand even if this figure only shows two compartments and one separation module, the invention is not limited thereto.

Upon activation, the system is adapted to suppress said separation provided by the separation module, thereby permitting mixing of the substances disposed in each compartment, here a tooth whitening agent and an additional substance.

In order to achieve the activation, the separation module separating at least two compartments can be a separation membrane adapted to be torn upon biting or a mobile languet, flexible or not, which can be moved by the user to reversibly create a communication between the compartments.

According to an even more preferred embodiment, such a separation module can be connected to a stirrer located in the system and adapted to stir the reagents contained of said at least two compartments upon displacement of said languet through some dedicated mechanism. The substances can therefore be stirred upon activation when they are mixed, thereby accelerating the mixing. The stirrer can have any suitable form such as a helix, spiral or a worm screw.

As is represented in figure 1 as well, the dental tray may further comprise at least one of alternative and/or complementary warming module such as an ultrasonic warming module, a light warming module and an electric warming module. Figure 1 shows LEDS acting as light warming module.

Figure 2, shows a second embodiment where the tooth whitening agent is PAP 21 and the where the compartments are not stacked but where the warming agent 22 is disposed around the PAP in the central compartment.

The self-warming composition is the product of an exothermic reaction. Preferably, the reagents of said exothermic reaction are contained in at least two separated compartments of said tooth whitening composition delivery system. An example of self-warming composition is sodium acetate or a mixture of vinegar and sodium bicarbonate or the same.

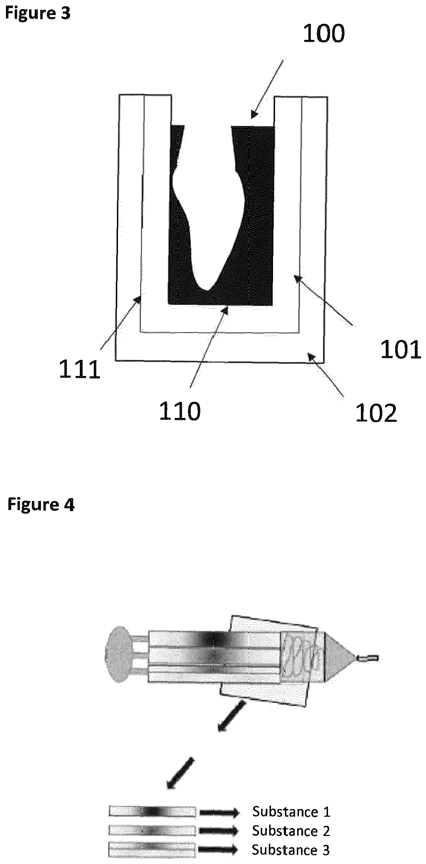

Figure 3 shows a cross section of a tooth whitening composition delivery system according to a third embodiment of the invention where we can see that the dental tray comprises three compartments, one for the whitening agent, one for a first self-warming reagent and one of a second warming reagent. The shown tooth is here to illustrate where the user is expected to bite and shall not limit the scope of this figure.

Preferably, the compartments are arranged so as to provide a predetermined sequence of mixing step. This can be done by providing separation modules with different resistances. For example in figure 3, the separation 111 is adapted to break before the separation 110 such that self-warming reagents 101 and 102 are mixed together before they are mixed with the tooth whitening agent 100.

Figure 4 shows a fourth embodiment where the delivery system is no longer a dental tray, but a syringe. Such a syringe presents four compartments and a mixing chamber located between the compartments and the outlet, such that a proper whitening gel comprising all the substances mixed can be obtained at the outlet. The activation consists in pushing the pushing element such that the separation is suppressed by pushing the substances out of the compartments towards the mixing chamber.

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This for example particularly the case regarding the number of compartments, the combination of additional substances and the combination of the different mentioned separation modules.

## Claims

1. Tooth whitening composition delivery system comprising a plurality of compartments adapted to house at least one tooth whitening agent and at least one additional substance chosen in the group comprising a self-warming composition and a doping substance, in different compartments, said compartments being separated by a separation module, wherein the system is adapted to suppress said separation provided by the separation module upon activation, thereby permitting mixing of the tooth whitening agent and the at least one additional substance.

2. Tooth whitening composition delivery system according to claim 1, **characterized in that** the at least one tooth whitening agent is selected in the group comprising H2O2, carbamide peroxide and PAP.

3. Tooth whitening composition delivery system to claim 1 or 2, **characterized in that** the doping substance is selected in the group comprising iron, chelated iron, and rejuvenating agents

4. Tooth whitening composition delivery system according to any one of claims 1 to 3, **characterized in that** the self-warming composition is the product of an exothermic reaction.

5. Tooth whitening composition delivery system according to claim 4, **characterized in that** the reagents of said exothermic reaction are contained in at least two separated compartments of said tooth whitening composition delivery system.

6. Tooth whitening composition delivery system according to any one of claims 1 to 5, **characterized in that** the self-warming composition comprises sodium acetate or a mixture of vinegar and sodium bicarbonate or the same.

7. Tooth whitening composition delivery system according to any one of claims 1 to 6, **characterized in that** it further houses at least one of sodium hyaluronate at concentrations of 5% or lower, Aloe Vera at concentrations of 5% or lower, sugar alcohols such as Xylitol, Sorbitol, Erythritol to the gel/liquid, cranberry seed oil at concentrations of 5% or lower, Chamomile at concentrations of 5% or lower, baking soda to the gel/liquid at concentrations of 5% or lower, and stable ozonized oils made out of unsaturated plant acids at concentrations of 5% or lower.

8. Tooth whitening composition delivery system according to any one of claims 1 to 7, **characterized in that** it further houses at least one of hyaluronic acid, remineralizing and/or desensibilizing agents such as CPP/ACP, amorphous calcium phosphate, potassium nitrate or fluoride.

9. Tooth whitening composition delivery system according to any one of claims 1 to 7, **characterized in that** the delivery system is a dental tray.

10. Tooth whitening composition delivery system according to claim 8, **characterized in that** activation is provided by biting of the dental tray.

11. Tooth whitening composition delivery system according to any one of claims 9 or 10, **characterized in that** the dental tray further comprises at least one of an ultrasonic warming module, a light warming module and an electric warming module.

12. Tooth whitening composition delivery system according to any one of claims 1 to 11, **characterized in that** the compartments are stacked on each other on the vertical direction, where vertical means the direction between the upper and lower jaws once the dental tray is in the biting position.

13. Tooth whitening composition delivery system according to any one of claims 1 to 12, **characterized in that** the compartments are arranged so as to provide a predetermined sequence of mixing step.

14. Tooth whitening composition delivery system according to claim 13, **characterized in that** the predetermined sequence of mixing step is achieved by providing separation modules with different resistances.

15. Tooth whitening composition delivery system according to any one of claims 1 to 14, **characterized in that** a separation module separating at least two compartments is at least one of a separation membrane adapted to be torn upon biting and of a mobile languet connected to a stirrer wherein the stirrer is adapted to stir the reagents contained of said at least two compartments upon displacement of said languet.

16. Tooth whitening composition delivery system according to claim 15, **characterized in that** the stirrer is at least one of a helix, spiral and a worm screw.

17. Tooth whitening composition delivery system according to any one of claims 1 to 8, **characterized in that** the delivery system is a syringe.
